# EUROPEAN PATENT APPLICATION

(11) **EP 3 751 487 A1**
(43) Date of publication of application: **16.12.2020**
(21) Application number: 19020391.9
(22) Date of filing: 19.06.2019
(51) Int. Cl.: G06Q 10/10

(54) **VIRTUAL ADVISORY BOARD**

(30) Priority: 12.06.2019 US 201916438679
(71) Applicant: Medical Pharma Services, s.r.o., 160 00 Prague 6 (CZ)
(72) Inventor: Denisova, Natalia, 160 00 Prague 6 (CZ)
(74) Representative: Halaxová, Eva

(57) **Abstract**

A system and method for providing a virtual advisory board includes receiving data related to the virtual advisory board, inviting potential participants, confirming actual participants, and tracking usage data for the actual participants. In further embodiments, the system and method may include setting a compensation amount for the actual participants. Related systems and methods for generating a report of a virtual advisory board are also included.

## Description

### TECHNICAL FIELD

The present invention relates to systems and methods for a virtual advisory board. The virtual advisory board may be specifically implemented with respect to medical advisory boards and provides a needed solution for the medical research and pharmaceutical industries. For example, the virtual advisory board may facilitate research and discussion by international experts in various therapeutic areas.

### BACKGROUND

A medical advisory board (also referred to as an advisory board or a board) is a group of recognized leading healthcare professionals who meet to discuss healthcare issues and solutions. The board is often headed by a chairperson who directs the meeting discussion. The discussion may include specific literature related to the pertinent healthcare issue as well as an agenda. Further, the discussion may result in a consensus opinion, proposed guidelines, a formal opinion, etc. regarding solutions to the pertinent healthcare issue.

The advisory board thus brings the experience and expertise of practicing healthcare professionals (HCPs) and key opinion leaders (KOLs) together with literature and research available in the field. Region-specific and practical solutions may then be determined.

In the pharmaceutical industry, medical advisory boards are often used as a reliable way to obtain insights for better understanding and interpretation of the medical benefits of pharmaceutical products. Research-focused companies may provide productive and transparent collaboration with external consultants in a manner where full integrity, fair remuneration, transparency, and confidentiality are maintained. Pharmaceutical companies an achieve the same benefits, but must adhere to stringent laws, regulations, industry codes, and company standards. Proper advisory boards must be driven by a specific and legitimate research, such as soliciting and obtaining knowledge-enhancing information and advice based on a genuine scientific/medical or business need.

Contrarily, when advisory boards are improperly run, there can be real or perceived improper relationship building, inducement to prescribe, recommend, purchase, supply or administer a medicinal product, or a disguised promotional activity (for example, improper off-label or pre-approval promotion). Further, questions may arise about the legitimacy of any payment provided.

Still further, advisory board meetings may be time consuming and logistically difficult. For example, it may be difficult to coordinate a location suitable for all attendees and their itinerary without violating industry codes related to hospitality standards, laws, regulations, etc. Unexpected cancellations and delays must be dealt with. Further, even if all attendees are present, the discussion itself must be moderated, with relevant information and literature provided. Sufficient time to review, discuss, etc. must be allocated. Further, appropriate confidentiality must always be maintained.

When all expected attendees are present, there is the cost for travel, meals, lodging, etc. Further, there are also costs to the participants practices and patients given that the participant is not available to conduct their normal practice and provide healthcare services.

Thus, it will be appreciated that there are many difficulties and expenses (monetary and time-related) to be dealt with in typical medical advisory boards. Systems and methods for reducing these difficulties and expenses while providing a state-of-the-art virtual advisory board would be well received in the industry.

### SUMMARY

An embodiment of the present invention relates to a method, and associated computer system and computer program product, for providing a virtual advisory board. A processor receives data related to the virtual advisory board. The processor invites a plurality of potential participants to join the virtual advisory board and confirms attendance of actual participants who have joined the virtual advisory board. The processor further presents at least one vote or survey to the actual participants, and records responses from the actual participants. The processor tracks usage data for the actual participants and sets a compensation amount for each actual participant.

Another embodiment of the present invention relates to a method, and associated computer system and computer program product, for generating a report for a virtual advisory board. A processor receives data. The processor invites a plurality of potential participants to join the virtual advisory board and confirms attendance of actual participants who have joined the virtual advisory board. The processor further provides actual participants with access to the data such that the actual participants are permitted to interact with the data and tracks usage data for the actual participants. The processor also presents at least one vote or survey related to the data to the actual participants and records responses from the actual participants. Then the processor generates the final report for the virtual advisory board.

### BRIEF DESCRIPTION OF THE DRAWINGS

Some of the embodiments will be described in detail, with reference to the following figures, wherein like designations denote like members, wherein:
FIG. 1 depicts a block diagram of a virtual advisory board system in accordance with embodiments of the present invention.
FIG. 2 depicts a block diagram of the virtual advisory board system in accordance with further embodiments of the present invention.
FIG. 3 depicts a flow chart of a method for providing a virtual advisory board, in accordance with embodiments of the present invention.
FIG. 4 depicts a flow chart of a method for providing a virtual advisory board, in accordance with further embodiments of the present invention.
FIG. 5 depicts a flow chart of a method for providing a virtual advisory board, in accordance with still further embodiments of the present invention.
FIG. 6 depicts a block diagram of a computer system for the virtual advisory board system of

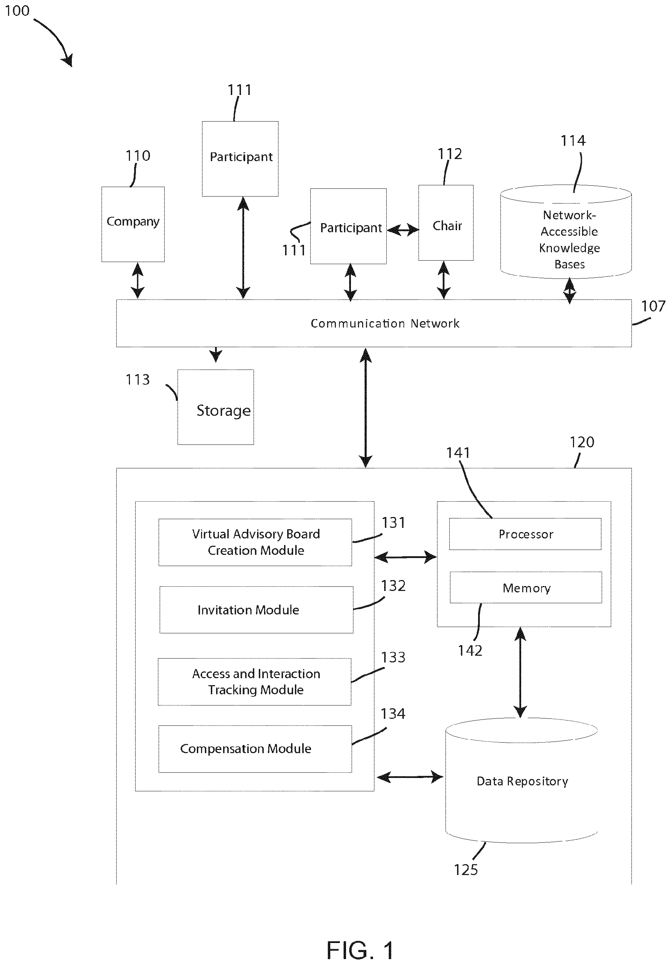

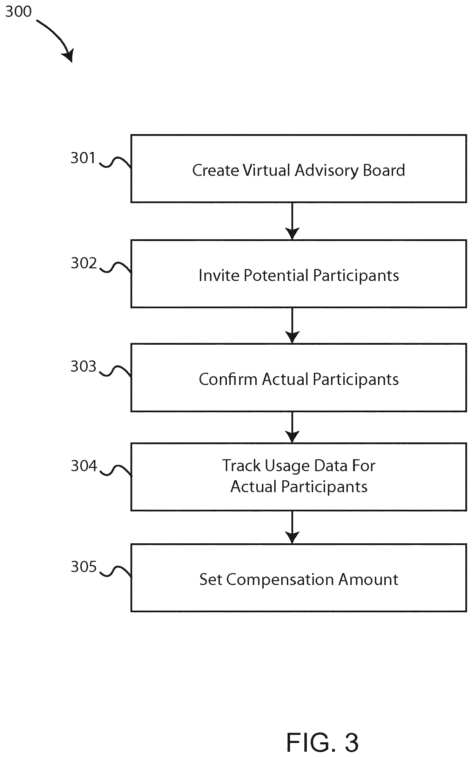

FIGS. 1-2, capable of implementing the methods of FIGS. 3-5, in accordance with embodiments of the present invention.

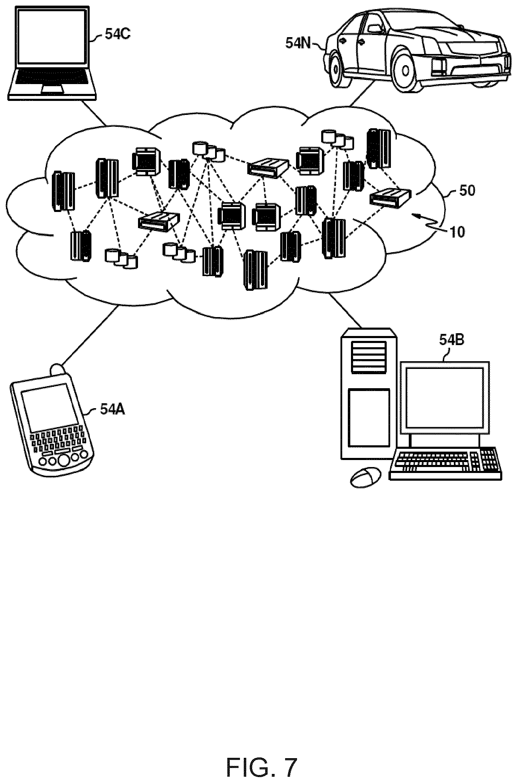

FIG. 7 depicts a cloud computing environment, in accordance with embodiments of the present invention.

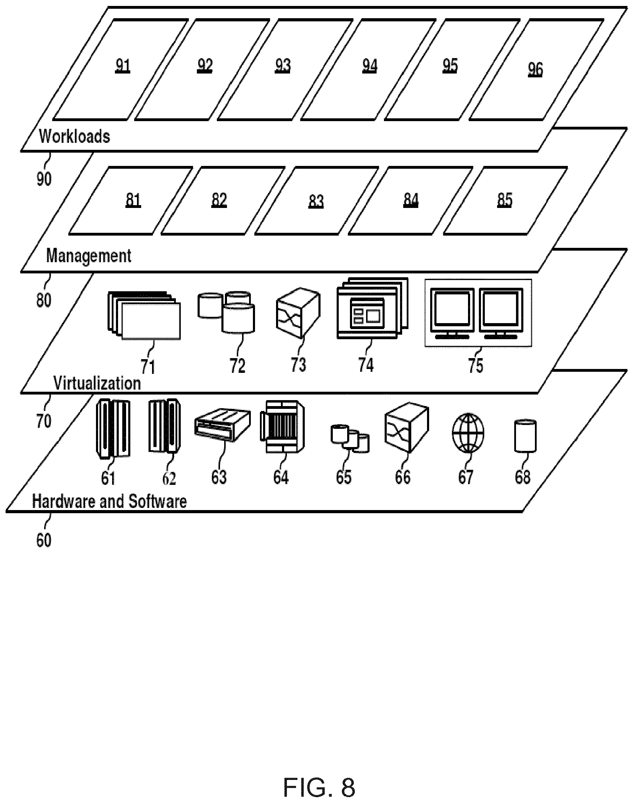

FIG. 8 depicts abstraction model layers, in accordance with embodiments of the present invention.

In the figures, like numbers refer to like objects throughout. Objects in the diagrams are not necessarily drawn to scale.

### DETAILED DESCRIPTION

In general, medical advisory boards are associated with logistical difficulties and costs as described above. Further, significant regulations, laws, codes, norms, and the like must be complied with in the medical and pharmaceutical industries.

As an improvement Applicant discloses a digital or virtual medical advisory board. Applicant's virtual medical advisory board provides the convenience of always being "open" and allowing practicing healthcare professionals and key opinion leaders to share input in an efficient, pressure-free manner, with no risk of last-minute cancellations and minimal logistical hurdles. Further, there will be a significant cost savings alongside increased adherence to all laws, regulations, codes, industry norms, etc. There is also the benefit for patients as their healthcare providers are not required to travel and thus be unavailable.

A detailed description of the hereinafter described embodiments of the disclosed apparatus and method are presented herein by way of exemplification and not limitation with reference to the Figures.

Referring now to the drawings, FIG. 1 depicts a block diagram of a virtual medical advisory board system 100 in accordance with embodiments of the invention. Embodiments of the virtual medical advisory board system 100 may be described as a system for providing, creating, managing, overseeing, and/or operating a virtual medical advisory board system.

In further embodiments, the virtual medical advisory board system 100 may also be a system for generating or providing a medical advisory board report or a medical advisory board resolution. The terms report and resolution are used interchangeably herein. Either or both of the final report and the resolution may be a document, report, or publication, or a combination of documents, reports, or publications that provide the findings or conclusions of the virtual medical advisory board. The generation of the report may be required within a certain time frame. For example, in some situations, the resolution is customarily required within 45 days. Further, in some embodiments, there may be a requirement that the report or resolution be signed by the chair. Further, as is discussed in more detail below, the report may be required to contain information related to all the participants in the advisory board as well as details regarding their interactions and contributions.

Turning back to FIG. 1, an embodiment of the virtual medical advisory board system 100 may comprise a plurality of participants 111 communicatively coupled to a computing device 120 over a communication network 107. The number of participants 111 connecting to the computing device 120 over the network 107 may vary in different embodiments. The participants 111 may be entities that would be invited to participate in a medical advisory board. For example, the participants 111 may be practicing healthcare professionals and/or key opinion leaders. As more detailed examples, the participants 111 may be experts, doctors, researchers, pharmacists, and the like. Further, one of the participants 111 may be designated as the chair or director-in FIG. 1, the chair or director is designated as 112. The chair 112 may be responsible for directing the discussion of the virtual advisory board. Further, the chair 112 may act as a mediator or moderator and may be responsible for ensuring that the virtual advisory board reaches a conclusion. For example, the chair 112 may work to try to get the participants 111 to reach an agreement on a recommendation or opinion.

The virtual medical advisory board system 100 may also comprise a company 110. The company 110 may be an entity requesting, initiating, or sponsoring the medical advisory board. For example, the company 110 may be a pharmaceutical company or similar entity looking for opinions of practicing healthcare professionals and/or key opinion leaders.

A plurality of companies 110 may be included in the system, although FIG. 1 only depicts a single company 110. It will be understood that a virtual medical advisory board is typically initiated on the system 100 by a single company 110. The single company 110 typically wishes to receive information and feedback from a plurality of participants 111 as discussed above. Further, despite the depiction of only a single company 110, it will be understood that multiple companies may each use the virtual medical advisory board system 100 to create one or more virtual advisory boards, either simultaneously or at various times.

The company 110 and/or participants 111 may connect to the communication network 107 and/or the computing device 120 in response to an invitation to join or by requesting to join. The connection may be made for the purposes of joining an existing virtual medical advisory board or with the intent of joining a virtual medical advisory board at some point in the future. The company 110 and/or the participants 111 may create an account with the virtual medical advisory board system 100. When the company 110 and/or the participants wish to use the virtual medical advisory board system 100, for example, wish to create a virtual advisory board or with to participate in a virtual advisory board, they may log into their respective account.

The accounts for the company 110 and/or participants 111 may include identifying information about the respective entities. For example, company 110 accounts may include name, address, products on the market, products in development, areas of focus, research areas, key employees and researchers, prior advisory boards created, and other information. Participant 111 accounts may include name, address, title, position, educational credentials, work experience, prior advisory boards participated in, usage data for prior advisory boards participated in, publications, research conducted, and other information.

To create an account on the virtual medical advisory board system 100, the company 110 and/or participants 111 may be required to enter into a contract. For example, the contract may be between the company 110 and/or the participants 111, on the one hand, and an entity operating the virtual medical advisory board system 100, on the other hand. The contract may also specify terms of the relationship between the company 110 and the participants. The contract may set terms of use, confidentiality restrictions, compensation terms, and other terms governing the relationship between entities as well as the operation of the virtual medical advisory board system 100.

The virtual medical advisory board system 100 may further comprise a storage device 113. The storage device 113 may store information received by the virtual medical advisory board system 100 system from the company 110, participants 111, chair 112, or other sources, for example, through the network 107. Further, the storage device 113 may be used to store any data generated or used by the virtual medical advisory board system 100.

The communication network 107 may refer to a group of two or more computer systems linked together. Communication network 107 may be any type of computer network known by individuals skilled in the art. Examples of computer networks 107 may include a LAN, WAN, campus area networks (CAN), home area networks (HAN), metropolitan area networks (MAN), an enterprise network, cloud computing network (either physical or virtual) e.g. the Internet, a cellular communication network such as GSM or CDMA network or a mobile communications data network. The architecture of the communication network 107 may be a peer-to-peer network in some embodiments, wherein in other embodiments, the network 107 may be organized as a client/server architecture.

In some embodiments, the network 107 may further comprise a connection to one or more network-accessible knowledge bases 114 such as network repository, which are network repositories containing information, network repositories or other systems connected to the network 107 that may be considered nodes of the network 107. In some embodiments, where the computing system 120 or network repositories allocate resources to be used by the other nodes of the network 107, the computing system 120 and network-accessible knowledge bases 114 may be referred to as servers.

The network-accessible knowledge bases 114 may be a data collection area on the network 107 which may back up and save all the data transmitted back and forth between the nodes of the network 107. For example, the network repository may be a data center saving and cataloging information sent between/among the company 110, the participants 111, the chair 112, the storage device 113, the computing system 120, etc., as well as any combinations thereof. In some embodiments, a data collection center housing the network-accessible knowledge bases 114 may include an analytic module capable of analyzing each piece of data being stored by the network-accessible knowledge bases 114. Further, the computing system 120 may be integrated with or as a part of the data collection center housing the network-accessible knowledge bases 114. In some alternative embodiments, the network-accessible knowledge bases 114 may be a local repository that is connected to the computing system 120.

It will be understood that the virtual medical advisory board system 100 may include a user interface (not depicted) as would be known in the art. The user interface may be used by the company 110, the participants 111, the chair 112, etc. For example, the user interface may comprise a webpage with login features, account features, and the like. Additional features may be accessed as discussed in more detail below, including but not limited to options for creating/initiating a virtual advisory board, accessing or interacting with data, interacting with other participants, and the like.

Referring again to FIG. 1, embodiments of the 100 are explained in more detail. In some embodiments, the computing device 120 may be a computing system that includes a virtual advisory board creation module 131, an invitation module 132, an access and interaction tracking module 133, and a compensation module 134. A "module" may refer to a hardware based module, software based module or a module may be a combination of hardware and software. Embodiments of hardware based modules may include self-contained components such as chipsets, specialized circuitry and one or more memory devices, while a software-based module may be part of a program code or linked to the program code containing specific programmed instructions, which may be loaded in the memory device of the energy metering device 120. A module (whether hardware, software, or a combination thereof) may be designed to implement or execute one or more particular functions or routines. The computing system 120 may also be equipped with a memory device 142 which may store various information and data regarding the company 110, the participants 111, the chair 112, information from the storage device 113, other information, etc., and a processor 141 for implementing the tasks associated with the virtual medical advisory board system 100.

Embodiments of the virtual advisory board creation module 131 may include one or more components of hardware and/or software program code for creating or initiating a virtual advisory board. The virtual advisory board may be requested by the company 110. For example, the company 110 may login to its account with the virtual medical advisory board system 100. The company 110 may then request that the virtual medical advisory board system 100 create a new virtual advisory board. In order to start the new virtual advisory board, the company 110 may provide a topic or issue it wishes to have discussed or commented on by medical professionals. For example, the topic or issue may be a medical condition, medical device, pharmaceutical composition or product, treatment method, test result, treatment outcome information, and the like. The topic may include multiple subtopics or may comprise multiple related topics.

In a further embodiment, the topic may be related to off-label drug information or pre-approval information. Such topics may be governed by medical affairs accountability and responsibility restrictions. In some embodiments, these restrictions may have been already agreed to by the participants 111, or example, they may have been specified in the contract. In further embodiments, separate agreements may be used for the specific virtual advisory board.

At the initiation of the new virtual advisory board, the company 110 may provide data related to the topic or issue. For example, the data related to the topic or issue may include an overview of the issue, an agenda for questions to be discussed/answered, medical literature related to the problem, and the like. In the case of test result or treatment outcome information, the data related to the topic may be the test result or treatment outcome information itself. Still further, the data may be or may be related to a pharmaceutical product, a medical device, a medical condition, a medical procedure, a treatment method, a diagnostic method, and the like.

The data may also include user guidance information. User guidance information may comprise information related to using the virtual medical advisory board system 100, navigating the user interface, additional details regarding the topic or the agenda, explanations of specific discussions desired by the company 110, and the like.

The data related to the topic may be stored in the storage device 113 or in the computing system 120 itself, for example, in the memory device 142.

Embodiments of the invitation module 132 may include one or more components of hardware and/or software program code for inviting participants 111 to join the virtual advisory board created by the virtual advisory board creation module 131. For example, the invitation module 132 may send invitations to participants 111 who have accounts with the virtual medical advisory board system 100. Additionally or alternatively, the invitation module 132 may be configured to send invitations to participants 111 who do not yet have accounts, for example, by emailing invitations to or using other contact means. In some embodiments, the invitation module 132 may only invite participants identified by the company 110 as candidates whose input would be relevant, helpful, or desired for the topic or issue. In other embodiments, the invitation module 132 may be configured to suggest participants 111 to the company 110 or to itself invite participants 111. In further embodiments, the virtual medical advisory board system 100 may include additional actors, for example, employees or experts for identifying and inviting participants 111. In some embodiments, the final decision on whether a participant 111 is included in the virtual advisory board may be controlled by the chair 112 or by the company 110. Invitations may be determined by matching the company 110 account information to the participant 111 information to determine likely candidates. Additionally or alternatively, participant 111 information may be matched to the issue or topic of the virtual advisory board and participants 111 may be chosen or suggested to the company 110. Further, if allowed by the company 110 initiating the virtual advisory board, the virtual advisory board may be made public, or a title, summary, or description of the virtual advisory board may be made public, so that participants 111 may be able to review virtual advisory boards and apply for participation. Still further, in some embodiments, the chair 112 may be in charge of inviting participants 111, reviewing suggested or invited participants 111, or otherwise overseeing the invitation process.

Participants 111 who are invited are then given the option to formally join the virtual advisory board. It will be understood that login procedures may be used to control the creation of the virtual advisory board as well as the acceptance of invitations. For example, a company 110 may be required to login with a password to create a virtual advisory board. The password may again be required to formally create the virtual advisory board. Likewise, participants 111 may be required to enter a password to login, to accept an invitation to an advisory board, or to access an advisory board after they have joined.

Participants 111 who are invited and actually join the virtual advisory board may be referred to as actual participants. In some embodiments, the company 110 or the chair 112 may need to provide a further approval, verification, or confirmation in order for a participant to join and become an actual participant.

Actual participants may be required to sign or otherwise accept or acknowledge a non-disclosure form, a confidentiality agreement, or the like, in which the actual participant agrees not to share or disseminate information received or accessed through the virtual medical advisory board system 100 or as part of the virtual advisory board. Given the proprietary nature of the issues/topics to be discussed in the virtual advisory boards, the acceptance/acknowledgement may be required for a participant to join and become an actual participant. In some embodiments, this acceptance may be included as part of the contract discussed above or may be a separate document.

Actual participants may access the data associated with the virtual advisory board and may interact with the data. Actual participants may be allowed to access and interact with the data using the user interface as described above. For example, actual participants may be permitted to review the data, comment on the data, edit the data, add new data, and the like. Further, actual participants may be allowed to interact with other actual participants, for example by using features such as a forum feature, a chat feature, and the like. Still further, actual participants may "like", "comment" on, or otherwise interact with material from other actual participants. In some embodiments, the interaction with the data may occur by voice or video interactions, for example, participants may engage in debates, comments, suggestions, discussions, and the like. Such interactions may occur in real time or over a period of time. For example, an actual participant may comment on the data using video or a voice recording. Another participant may respond using the same medium or may comment, like, or otherwise respond. In still further embodiments, multiple participants may discuss via voice and/or video in real time. In a further embodiment, the user interface may have an editable document feature in which the actual participants' edits are shown to other participants and in which other actual participants can comment, like, reject, accept, or alter the edits or take other action.

The virtual medical advisory board system 100 may notify actual participants when actions have been taken, for example, when information has been added or when other actual participants have interacted in some way. The notifications may be sent by email, text, SMS messaging, within the system itself, or in other ways. Actual participants may have the option to change notification settings, or the settings may be set by the company 110, the chair 112, or the virtual medical advisory board system 100.

In some embodiments, the company 110 may be allowed to participate as well. For example, the company 110 or a participant associated with the company 110 may be allowed to take the same actions permitted for the actual participants. Alternatively, the company 110 or a participant associated with the company may be limited to viewing the actions taken by the actual participants, for example, being able to access or read the comments and discussion, but not being able to comment, like, edit, etc.

Embodiments of the access and interaction tracking module 133 may include one or more components of hardware and/or software program code for tracking, recording, and/or monitoring access and interaction of the actual participants. The tracked, recorded, and/or monitored access and interaction may be broadly referred to as usage data. For example, in some embodiments, usage data may include the amount of time the actual participant is logged onto the virtual advisory board, i.e., login duration. The access and interaction tracking module 133 may also track login times. The access and tracking interaction module 133 may also track access to and/or interaction with the data, for example, the types of actions discussed above. The access and interaction tracking module may also monitor interactions with other actual participants regarding the issue or topic, the data, or other information pertaining to the virtual advisory board. The access and tracking interaction module 133 may also track the voice or video interactions described above. For example, debates, comments, suggestions, discussions and the like by one or more actual participants which occur may be recorded and tracked as usage data.

Still further, the usage data for an actual participant may include tracking of other actual participants' interactions with information or material added or provided by an actual participant. For example, if a first participant provides research data relevant to the topic/issue, and other actual participants comment/discuss or otherwise interact with the research data, such interactions may be tracked with respect to the first participant as well as the other actual participants who then interact with the research data. Likewise, if a first participant provides a comment that generates a substantial discussion or substantial follow up comments, the additional interactions may be tracked as usage data for the first participant.

Thus, usage data may be used to provide an approximation of the amount of time an actual participant devotes to the virtual advisory board and/or as an approximation of the contribution the actual participant adds to the virtual advisory board. The usage data may also provide an approximation of the value provided by the actual participant.

An additional feature of the virtual medical advisory board system 100 is the ability of the company 110 or the chair 112 to generate surveys, polls, questions, tests, quizzes, votes, etc. These may be presented to the actual participants, either for further discussion or simply for collecting responses. For example, a survey may ask a question related to the topic or issue, or to a tangential issue, and provide multiple choice type response options. Alternatively, the actual participant may be able to create their own unique response. The results/answers/responses are recorded by the virtual medical advisory board system 100 and may be presented to the company 110 directly. Additionally or alternatively, the results/answers/responses may be monitored by the access and interaction tracking module as yet another type of usage data.

The access and interaction tracking module 133 may keep metrics related to usage data, for example, a "running" "score" of each actual participant's usage data. For example, the access and interaction tracking module 133 may track total or absolute values for participation, relative values (i.e., compared to a benchmark, a historic average, an average of all current actual participants, and the like), and similar metrics. The metrics may be kept confidential, may be displayed to just the respective active participant, to the company 110, to the chair 112, to all actual participants, or a combination thereof. Further, the company 110 or the chair 112 may control whether the metrics are kept confidential or are shared/displayed.

Embodiments of the compensation module 134 may include one or more components of hardware and/or software program code for determining a compensation amount for the respective actual participants. The compensation amount may be at least partially based on the tracked usage data, i.e., the amount of time or participation the actual participant provides to the virtual advisory board. Still further, the compensation amount may be at least partially based on the actual participant's account information as discussed above, for example, the actual participant's address, title, position, educational credentials, work experience, prior advisory boards participated in, publications, research conducted, salary, and other information. For example, the compensation amount may be determined such that it is commensurate with fair market value for the actual participant's time, considering the actual participant's location, title or position, skill, expertise, experience, and the like. Still further, the compensation amount may be at least partially based on the tracked usage data for the respective actual participant. For example, the compensation amount may be determined such that it accounts for the value the actual participant provided to the virtual advisory board, for example, the amount of discussion, the amount of feedback provided to others, the information provided/added, and the like. Thus, in some embodiments, compensation amounts may be determined for the actual participants such that actual participants with higher usage data are compensated at higher rates or with higher amounts than actual participants with lower usage data. As an example, in the situation given above wherein a first actual participant provided research data relevant to the topic, the additional interactions of other actual participants related to the provided research data may be attributed to the first actual participant as well as the other participants interacting with the research data. Thus, the first actual participant's tracked usage data may be increased to reflect the contribution to the virtual advisory board and to compensate the participant accordingly.

The compensation amount may also be based at least partially on the contract, whether between the participant 111 and the company 110 or between the participant 111 and the virtual medical advisory board system 100. Further any combination of the listed bases for the compensation amount may be used and other additional factors may be applied as well.

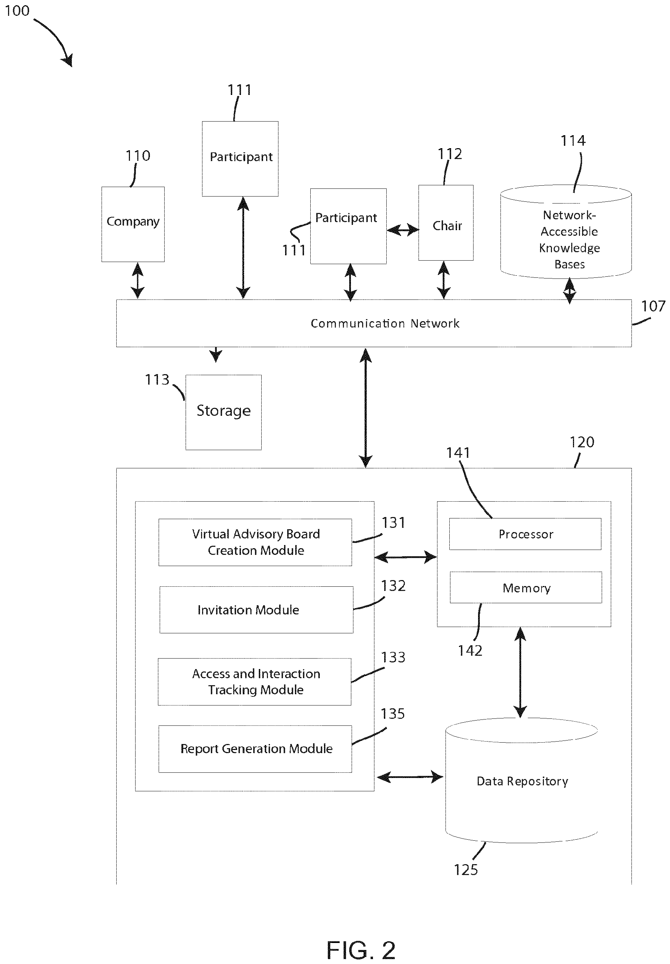

Turning now to FIG. 2, FIG. 2 depicts a block diagram of the virtual medical advisory board system 100 in accordance with further embodiments of the present invention, wherein the system further comprises a report generating module 135. As shown, this embodiment does not include the compensation module 134; however, in some embodiments, both the report generating module 135 and the compensation module 134 may be included.

The report generating module 135 may compile all of the data related to the virtual advisory board as well as the tracked usage data. For example, all of the access and interactions of the actual participants may be compiled along with any material added by the actual participants. Still further, the login times, login duration, etc. of the individual actual participants may also be compiled. The report generating module 135 may also compile voice recordings of the actual participants by transcribing the voice recordings in text format. The report generating module 135 may package this information into a final report or resolution as described above. The information may be provided to the chair for review and/or may be provided directly to the company. In some embodiments, the final report may include a resolution of the virtual advisory board, for example, a conclusion, a verdict, a recommendation, a suggestion, and the like related to the topic or issue. Still further, the resolution may be specifically related to the poll or survey presented to the actual participants, if one was presented.

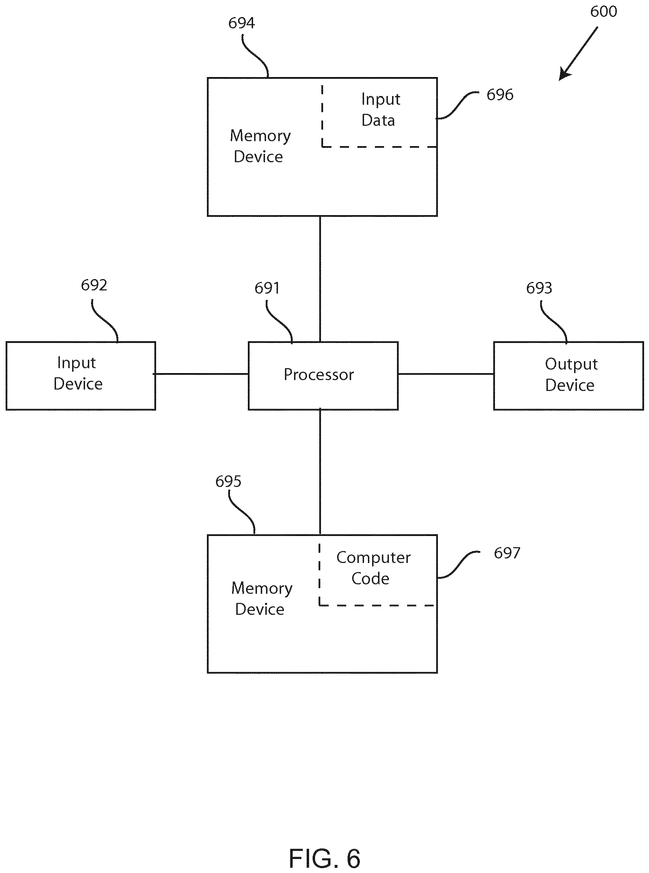

FIG. 3 depicts a flowchart showing a method of providing a virtual advisory board in accordance with embodiments of the present invention. One embodiment of a method 300 or algorithm that may be implemented for providing a virtual advisory board in accordance with virtual medical advisory board system 100 is described in FIG. 1 using one or more computer systems as defined generically in FIG. 6 below.

Embodiments of the method 300 for providing a virtual advisory board may begin at a step 301 wherein a new virtual advisory board is created, for example by the virtual advisory board creation module 131. The new virtual advisory board may be created in response to a request or command for a new virtual advisory board from a company, for example, the company 110. The company may join the virtual medical advisory board system 100 by creating an account or may have already had an account with the system.

The company may provide a title for the new virtual advisory board, as well as one or more of an agenda for the new virtual advisory board, an objective for the new virtual advisory board, and medical literature pertinent to the new virtual advisory board. As discussed above, the new virtual advisory board may be directed to a topic or issue, such as a medical condition, a pharmaceutical composition, compound, or ingredient, a treatment method, test result, and the like. Additional information may include information related to the company, such as account information or background information. This information and/or data may be received by the virtual medical advisory board system 100, for example by the computing unit 120, the processor 141, the virtual advisory board creation module 131, or a combination thereof.

Step 302 includes inviting a plurality of potential participants to joint the virtual advisory board created in step 301. The potential participants may be participants such as participants 111 who already have accounts on the virtual medical advisory board system 100 or the virtual medical advisory board system 100 may invite individuals to join the system. As discussed above, the potential participants may be chosen by the company 110 or by the virtual medical advisory board system 100. For example, the invitation module 132 may identify potential participants and invite them directly or may suggest or recommend them to the company 110 as potential participants. Still further, the chair 112 may be involved in determining which potential participants are invited to join the virtual advisory board.

Step 303 includes confirming actual participants. As discussed above, actual participants refer to participants who were invited and joined the created virtual advisory board. Still further, in some embodiments confidentiality agreements or forms must be executed in order for a participant to join the virtual advisory board and become an actual participant.

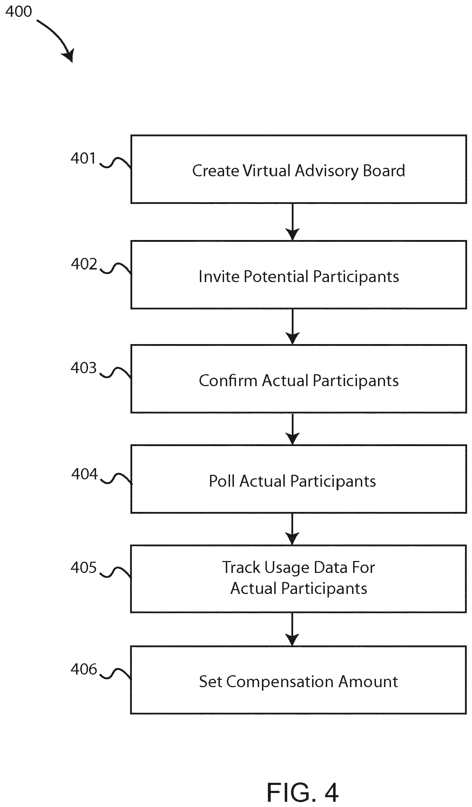

As is also discussed above, confirmed actual participants are allowed to view, review, access, and interact with the data related to the virtual advisory board, for example, the agenda, the objective, the medical literature, and any other data. Thus, the virtual advisory board may proceed and approximate a "real-life" or "in-person" advisory board. For example, the participants may have discussions or debates, may provide additional data for other actual participants to review, may share insights, may make recommendations, may make suggestions, may ask questions, etc. For example, actual participants may share clinical cases, patient data, diagnostic materials or results, and the like. Confirmed actual participants may also have access to a list of actual participants and at least some of their account information as well as information regarding the company. Again, all the information may be shared confidentially based on the parameters of the confidentiality forms signed when joining the system 100 or the specific virtual advisory board. Still further, as discussed above, the company or actual participants may create a vote, survey, poll or the like in order to receive feedback from other actual participants or to otherwise advance the virtual advisory board. Results may be recorded. Results may be made available to the actual participants or may be restricted to the chair or to the company. In this embodiment, the activities of confirmed actual participants are not designated as a separate step; however, as shown in FIG. 4, in some embodiments, they may be unique method steps. For example, in FIG. 4 steps 401, 402, and 403 may be the same as steps 301, 302, and 303, respectively, while an additional step 404 including polling or surveying the actual participants is included. The method 400 of FIG. 4 is described in more detail below.

Step 304 includes tracking usage data for the actual participants. This tracking may refer to monitoring, analyzing, recording, and the like conducted with respect to the actual participants' access and interaction with the data related to the virtual advisory board, with other actual participants, and with additional information added by other participants. As discussed in more detail above, usage data may refer to login duration, login times, and specific actions/activities undertaken while logged in. Still further, the usage data may include responses from other actual participants to information/material provided by a first actual participant as discussed above. Participation in polls, votes, and surveys may also be included in the tracked usage data as has been described. Additionally, voice recordings of actual participants engaging in discussions or debates via voice and/or video interactions may be recorded and tracked as usage data. As discussed above, tracked usage data may provide an approximation of the time, value, contribution, etc. provided to the virtual advisory board by the respective actual participant.

Step 305 includes setting a compensation amount for the actual participants. The compensation amount may be set by the compensation module 134. As discussed above, the compensation amount may be set based at least partially on the account information for the respective actual participant. Using this account information, the compensation amount may be set so as to be commensurate with hourly rates for the actual participant, either in the actual participant's practice or in similar advisory boards (whether in person or virtual). Still further, the compensation may be set based at least partially on the usage data for the actual participant. Using the usage data, the compensation amount may be set so as to compensate each of the actual participants according to their respective contribution to the virtual advisory board, whether in terms of time, discussion value, or other characteristics. The compensation amount may also be set by contract or other agreement. A combination of these bases may be used, as may additional information.

Turning now to FIG. 4, depicted is a flowchart showing a method of providing a virtual advisory board in accordance with embodiments of the present invention. One embodiment of a method 400 or algorithm that may be implemented for providing a virtual advisory board in accordance with virtual medical advisory board system 100 is described in FIG. 1 using one or more computer systems as defined generically in FIG. 6 below.

Embodiments of the method 400 for providing a virtual advisory board may begin at a step 401 where a new virtual advisory board is created or initiated, and may continue to step 402 where potential participants are invited, and to step 403 where actual participants are confirmed. Each of these steps may proceed more or less as described above with respect to FIG. 3 and method 300.

Step 404 may include polling the actual participants. For example, a survey may be provided in which one or more questions related to the topic or issue of the virtual advisory board are presented. The actual participants may be instructed to choose an answer from a provided list of options or to create their own answer. In addition to or as an alternative to polling, in some embodiments, step 404 may include providing a document. For example, the document may include blank sections for the actual participants to input responses or information or may simply be a statement to which the actual participant is asked to agree or disagree. Still further, the document may be editable such that the actual participant may edit portions that the actual participant believes are incorrect, deficient, or need to be revised, updated, or changed based on their practice and experience. The poll, survey, or other document may be created by the company, a participant associated with the company, the chair, or by an actual participant. Still further, the virtual medical advisory board system 100 and/or the computing system 120 may create the poll, survey, or other document. For example, the system 100 may detect a disagreement between actual participants and present the poll, survey, or other document to obtain reliable information on the actual participants' opinions. Still further, the system 100 may detect that the actual participants have come to agreement on the topic/issue or a subtopic thereof and may use the poll, survey, or other document to confirm that a resolution has been reached. In either case, the poll, survey, or other document, and/or the results thereof may be used to generate the resolution or report as is described above.

Step 405 may include tracking usage data as has been described herein. The usage data may include the results of the polling/surveying conducted in step 404. This step may proceed more or less as described above with respect to FIG. 3 and method 300.

Step 406 may include setting a compensation amount. This step may proceed more or less as described above with respect to FIG. 3 and method 300.

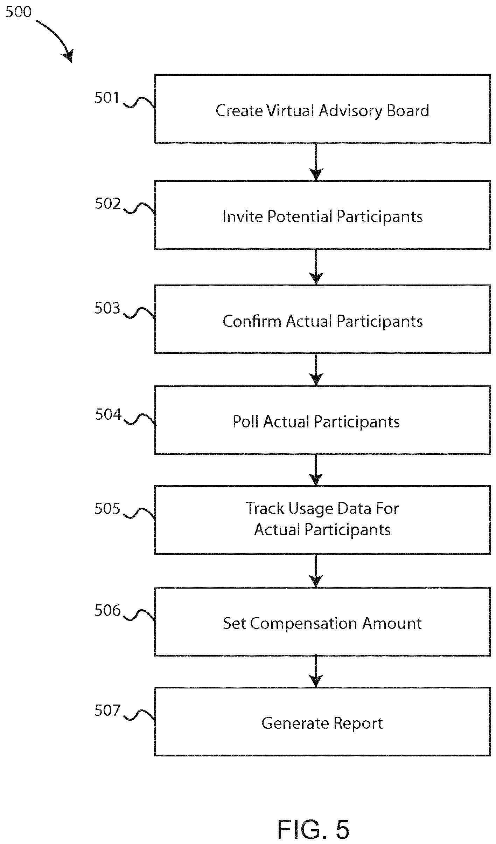

Turning now to FIG. 5, depicted is a flowchart showing a method of providing a virtual advisory board in accordance with embodiments of the present invention. The method may also be referred to as a method of generating a report for a virtual advisory board. One embodiment of a method 500 or algorithm that may be implemented for providing a virtual advisory board in accordance with virtual medical advisory board system 100 is described in FIG. 1 using one or more computer systems as defined generically in FIG. 6 below.

Embodiments of the method 500 for providing a virtual advisory board or for generating a report for a virtual advisory board may begin at a step 501 where a new virtual advisory board is created or initiated, and may continue to step 502 where potential participants are invited, to step 503 where actual participants are confirmed, step 504 where actual participants are polled, and step 505 where usage data for actual participants is tracked. Each of these steps may proceed more or less as described above with respect to FIGS. 3 and 4 and methods 300 and 400. It will be understood that step 504 may be an optional step as shown in the differences between method 300 and method 400. Further, step 506 may be optionally included as shown, although in some embodiments the step of setting the compensation amount may not be used.

The method 500 may include step 507 where a report is generated for the virtual advisory board. In some embodiments, this step may be conducted by the reporting generation module 135 discussed above. Further, as discussed above, step 507 for generating the report may include compiling the data related to the virtual advisory board along with the usage data for the actual participants. Still further, step 507 may utilize the results of the poll, survey, or other document as described above. Additionally, step 507 may include transcribing voice and/or video recordings of actual participants and converting the recordings into text form. The report or resolution may be presented to the chair to be verified and signed. Further, the report may be saved along with the underlying data and information.

While a termination step is not depicted, it will be understood that the virtual advisory board may be terminated, for example, after the generation of the report or resolution or after compensation has been set or provided. All information and data may be saved and preserved for an appropriate amount of time. Further, the saved information and data may be provided to the company for their use and evaluation. Some data may be stored for later use by the virtual medical advisory board system 100, for example, for later use in tracking usage data or setting relative values for usage data. Confidential or proprietary information may be removed, scrubbed, deidentified, and the like as appropriate.

FIG. 6 depicts a block diagram of a computer system for the virtual medical advisory board system 100 of FIGS. 1-2, capable of implementing methods for providing a virtual advisory board and/or generating a report of a virtual advisory board of FIGS. 3-5, in accordance with embodiments of the present invention. The computer system 600 may generally comprise a processor 691, an input device 692 coupled to the processor 691, an output device 693 coupled to the processor 691, and memory devices 694 and 695 each coupled to the processor 691. The input device 692, output device 693 and memory devices 694, 695 may each be coupled to the processor 691 via a bus. Processor 691 may perform computations and control the functions of computer system 600, including executing instructions included in the computer code 697 for the tools and programs capable of implementing a method for providing a virtual advisory board and/or generating a report of a virtual advisory board of FIGS. 3-5 using the computer system for the virtual medical advisory board system 100 of FIGS. 1-2,, wherein the instructions of the computer code 697 may be executed by processor 691 via memory device 695. The computer code 697 may include software or program instructions that may implement one or more algorithms for implementing the method for providing a virtual advisory board and/or generating a report of an advisory board as described in detail above. The processor 691 executes the computer code 697. Processor 691 may include a single processing unit, or may be distributed across one or more processing units in one or more locations (e.g., on a client and server).

The memory device 694 may include input data 696. The input data 696 includes any inputs required by the computer code 697. The output device 693 displays output from the computer code 697. Either or both memory devices 694 and 695 may be used as a computer usable storage medium (or program storage device) having a computer-readable program embodied therein and/or having other data stored therein, wherein the computer-readable program comprises the computer code 697. Generally, a computer program product (or, alternatively, an article of manufacture) of the computer system 600 may comprise said computer usable storage medium (or said program storage device).

Memory devices 694, 695 include any known computer-readable storage medium, including those described in detail below. In one embodiment, cache memory elements of memory devices 694, 695 may provide temporary storage of at least some program code (e.g., computer code 697) in order to reduce the number of times code must be retrieved from bulk storage while instructions of the computer code 697 are applied. Moreover, similar to processor 691, memory devices 694, 695 may reside at a single physical location, including one or more types of data storage, or be distributed across a plurality of physical systems in various forms. Further, memory devices 694, 695 can include data distributed across, for example, a local area network (LAN) or a wide area network (WAN). Further, memory devices 694, 695 may include an operating system (not shown) and may include other systems not shown in FIG. 6.

In some embodiments, the computer system 600 may further be coupled to an Input/output (I/O) interface and a computer data storage unit. An I/O interface may include any system for exchanging information to or from an input device 692 or output device 693. The input device 692 may be, inter alia, a keyboard, a mouse, etc. or in some embodiments the touchscreen of a computing device. The output device 693 may be, inter alia, a printer, a plotter, a display device (such as a computer screen), a magnetic tape, a removable hard disk, a floppy disk, etc. The memory devices 694 and 695 may be, inter alia, a hard disk, a floppy disk, a magnetic tape, an optical storage such as a compact disc (CD) or a digital video disc (DVD), a dynamic random access memory (DRAM), a read-only memory (ROM), etc. The bus may provide a communication link between each of the components in computer 600, and may include any type of transmission link, including electrical, optical, wireless, etc.

An I/O interface may allow computer system 600 to store information (e.g., data or program instructions such as program code 697) on and retrieve the information from computer data storage unit (not shown). Computer data storage unit includes a known computer-readable storage medium, which is described below. In one embodiment, computer data storage unit may be a non-volatile data storage device, such as a magnetic disk drive (i.e., hard disk drive) or an optical disc drive (e.g., a CD-ROM drive which receives a CD-ROM disk). In other embodiments, the data storage unit may include a knowledge base or data repository 125 as shown in FIG. 1.

As will be appreciated by one skilled in the art, in one embodiment, the present invention may be a method; in a further embodiment, the present invention may be a system; and in a still further embodiment, the present invention may be a computer program product. Any of the components of the embodiments of the present invention can be deployed, managed, serviced, etc. by a service provider that offers to deploy or integrate computing infrastructure with respect to virtual advisory board systems and methods. Thus, an embodiment of the present invention discloses a process for supporting computer infrastructure, where the process includes providing at least one support service for at least one of integrating, hosting, maintaining and deploying computer-readable code (e.g., program code 697) in a computer system (e.g., computer system 600) including one or more processor(s) 691, wherein the processor(s) carry out instructions contained in the computer code 697 causing the computer system to provide virtual advisory boards and/or generate reports for virtual advisory boards. Another embodiment discloses a process for supporting computer infrastructure, where the process includes integrating computer-readable program code into a computer system 600 including a processor.

The step of integrating includes storing the program code in a computer-readable storage device of the computer system 600 through use of the processor. The program code, upon being executed by the processor, implements a method for providing a virtual advisory board and/or generating a report for a virtual advisory board. Thus, the present invention discloses a process for supporting, deploying and/or integrating computer infrastructure, integrating, hosting, maintaining, and deploying computer-readable code into the computer system 600, wherein the code in combination with the computer system 600 is capable of performing a method for providing a virtual advisory board and/or generating a report for a virtual advisory board.

A computer program product of the present invention comprises one or more computer-readable hardware storage devices having computer-readable program code stored therein, said program code containing instructions executable by one or more processors of a computer system to implement the methods of the present invention.

A computer system of the present invention comprises one or more processors, one or more memories, and one or more computer-readable hardware storage devices, said one or more hardware storage devices containing program code executable by the one or more processors via the one or more memories to implement the methods of the present invention.

The present invention may be a system, a method, and/or a computer program product at any possible technical detail level of integration. The computer program product may include a computer-readable storage medium (or media) having computer-readable program instructions thereon for causing a processor to carry out aspects of the present invention.

The computer-readable storage medium can be a tangible device that can retain and store instructions for use by an instruction execution device. The computer-readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer-readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer-readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer-readable program instructions described herein can be downloaded to respective computing/processing devices from a computer-readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer-readable program instructions from the network and forwards the computer-readable program instructions for storage in a computer-readable storage medium within the respective computing/processing device.

Computer-readable program instructions for carrying out operations of the present invention may be assembler instructions, instruction-set-architecture (ISA) instructions, machine instructions, machine-dependent instructions, microcode, firmware instructions, state-setting data, configuration data for integrated circuitry, or either source code or object code written in any combination of one or more programming languages, including an object oriented programming language such as Smalltalk, C++, or the like, and procedural programming languages, such as the "C" programming language or similar programming languages. The computer-readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer-readable program instructions by utilizing state information of the computer-readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer-readable program instructions.

These computer-readable program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer-readable program instructions may also be stored in a computer-readable storage medium that can direct a computer, a programmable data processing apparatus, and/or other devices to function in a particular manner, such that the computer-readable storage medium having instructions stored therein comprises an article of manufacture including instructions which implement aspects of the function/act specified in the flowchart and/or block diagram block or blocks.

The computer-readable program instructions may also be loaded onto a computer, other programmable data processing apparatus, or other device to cause a series of operational steps to be performed on the computer, other programmable apparatus or other device to produce a computer-implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the blocks may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

It is to be understood that although this disclosure includes a detailed description on cloud computing, implementation of the teachings recited herein are not limited to a cloud computing environment. Rather, embodiments of the present invention are capable of being implemented in conjunction with any other type of computing environment now known or later developed.

Cloud computing is a model of service delivery for enabling convenient, on-demand network access to a shared pool of configurable computing resources (e.g., networks, network bandwidth, servers, processing, memory, storage, applications, virtual machines, and services) that can be rapidly provisioned and released with minimal management effort or interaction with a provider of the service. This cloud model may include at least five characteristics, at least three service models, and at least four deployment models.

Characteristics are as follows:
*On-demand self-service*: a cloud consumer can unilaterally provision computing capabilities, such as server time and network storage, as needed automatically without requiring human interaction with the service's provider.
*Broad network access*: capabilities are available over a network and accessed through standard mechanisms that promote use by heterogeneous thin or thick client platforms (e.g., mobile phones, laptops, and PDAs).
*Resource pooling*: the provider's computing resources are pooled to serve multiple consumers using a multi-tenant model, with different physical and virtual resources dynamically assigned and reassigned according to demand. There is a sense of location independence in that the consumer generally has no control or knowledge over the exact location of the provided resources but may be able to specify location at a higher level of abstraction (e.g., country, state, or datacenter).
*Rapid elasticity*: capabilities can be rapidly and elastically provisioned, in some cases automatically, to quickly scale out and rapidly released to quickly scale in. To the consumer, the capabilities available for provisioning often appear to be unlimited and can be purchased in any quantity at any time.
*Measured service*: cloud systems automatically control and optimize resource use by leveraging a metering capability at some level of abstraction appropriate to the type of service (e.g., storage, processing, bandwidth, and active user accounts). Resource usage can be monitored, controlled, and reported, providing transparency for both the provider and consumer of the utilized service.

Service Models are as follows:
*Software αs a Service (SaaS)*: the capability provided to the consumer is to use the provider's applications running on a cloud infrastructure. The applications are accessible from various client devices through a thin client interface such as a web browser (e.g., web-based e-mail). The consumer does not manage or control the underlying cloud infrastructure including network, servers, operating systems, storage, or even individual application capabilities, with the possible exception of limited user-specific application configuration settings.
*Platform as a Service (PaaS):* the capability provided to the consumer is to deploy onto the cloud infrastructure consumer-created or acquired applications created using programming languages and tools supported by the provider. The consumer does not manage or control the underlying cloud infrastructure including networks, servers, operating systems, or storage, but has control over the deployed applications and possibly application hosting environment configurations.
*Infrastructure as a Service (IaaS)*: the capability provided to the consumer is to provision processing, storage, networks, and other fundamental computing resources where the consumer is able to deploy and run arbitrary software, which can include operating systems and applications. The consumer does not manage or control the underlying cloud infrastructure but has control over operating systems, storage, deployed applications, and possibly limited control of select networking components (e.g., host firewalls).

Deployment Models are as follows:
*Private cloud*: the cloud infrastructure is operated solely for an organization. It may be managed by the organization or a third party and may exist on-premises or off-premises.
*Community cloud*: the cloud infrastructure is shared by several organizations and supports a specific community that has shared concerns (e.g., mission, security requirements, policy, and compliance considerations). It may be managed by the organizations or a third party and may exist on-premises or off-premises.
*Public cloud*: the cloud infrastructure is made available to the general public or a large industry group and is owned by an organization selling cloud services.
*Hybrid cloud*: the cloud infrastructure is a composition of two or more clouds (private, community, or public) that remain unique entities but are bound together by standardized or proprietary technology that enables data and application portability (e.g., cloud bursting for load-balancing between clouds).

A cloud computing environment is service oriented with a focus on statelessness, low coupling, modularity, and semantic interoperability. At the heart of cloud computing is an infrastructure that includes a network of interconnected nodes.

Referring now to FIG. 7, illustrative cloud computing environment 50 is depicted. As shown, cloud computing environment 50 includes one or more cloud computing nodes 10 with which local computing devices used by cloud consumers, such as, for example, personal digital assistant (PDA) or cellular telephone 54A, desktop computer 54B, laptop computer 54C, and/or automobile computer system 54N may communicate. Nodes 10 may communicate with one another. They may be grouped (not shown) physically or virtually, in one or more networks, such as Private, Community, Public, or Hybrid clouds as described hereinabove, or a combination thereof. This allows cloud computing environment 50 to offer infrastructure, platforms and/or software as services for which a cloud consumer does not need to maintain resources on a local computing device. It is understood that the types of computing devices 54A, 54B, 54C and 54N shown in FIG. 7 are intended to be illustrative only and that computing nodes 10 and cloud computing environment 50 can communicate with any type of computerized device over any type of network and/or network addressable connection (e.g., using a web browser).

Referring now to FIG. 8, a set of functional abstraction layers provided by cloud computing environment 50 (see FIG. 7) are shown. It should be understood in advance that the components, layers, and functions shown in FIG.8 are intended to be illustrative only and embodiments of the invention are not limited thereto. As depicted, the following layers and corresponding functions are provided:

Hardware and software layer 60 includes hardware and software components. Examples of hardware components include: mainframes 61; RISC (Reduced Instruction Set Computer) architecture based servers 62; servers 63; blade servers 64; storage devices 65; and networks and networking components 66. In some embodiments, software components include network application server software 67 and database software 68.

Virtualization layer 70 provides an abstraction layer from which the following examples of virtual entities may be provided: virtual servers 71; virtual storage 72; virtual networks 73, including virtual private networks; virtual applications and operating systems 74; and virtual clients 75.

In one example, management layer 80 may provide the functions described below. Resource provisioning 81 provides dynamic procurement of computing resources and other resources that are utilized to perform tasks within the cloud computing environment. Metering and Pricing 82 provide cost tracking as resources are utilized within the cloud computing environment, and billing or invoicing for consumption of these resources. In one example, these resources may include application software licenses. Security provides identity verification for cloud consumers and tasks, as well as protection for data and other resources. User portal 83 provides access to the cloud computing environment for consumers and system administrators. Service level management 84 provides cloud computing resource allocation and management such that required service levels are met. Service Level Agreement (SLA) planning and fulfillment 85 provides pre-arrangement for, and procurement of, cloud computing resources for which a future requirement is anticipated in accordance with an SLA.

Workloads layer 90 provides examples of functionality for which the cloud computing environment may be utilized. Examples of workloads and functions which may be provided from this layer include: mapping and navigation 91; software development and lifecycle management 92; virtual classroom education delivery 93; data analytics processing 94; transaction processing 95; and virtual advisory board providing and/or advisory report generating 96.

The descriptions of the various embodiments of the present invention have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

Elements of the embodiments have been introduced with either the articles "a" or "an." The articles are intended to mean that there are one or more of the elements. The terms "including" and "having" and their derivatives are intended to be inclusive such that there may be additional elements other than the elements listed. The conjunction "or" when used with a list of at least two terms is intended to mean any term or combination of terms. The terms "first" and "second" are used to distinguish elements and are not used to denote a particular order.

While the invention has been described in detail in connection with only a limited number of embodiments, it should be readily understood that the invention is not limited to such disclosed embodiments. Rather, the invention can be modified to incorporate any number of variations, alterations, substitutions or equivalent arrangements not heretofore described, but which are commensurate with the spirit and scope of the invention. Additionally, while various embodiments of the invention have been described, it is to be understood that aspects of the invention may include only some of the described embodiments. Accordingly, the invention is not to be seen as limited by the foregoing description, but is only limited by the scope of the appended claims.

## Claims

1. A method of providing a virtual advisory board, the method comprising:
receiving, by a processor of a computing system, data related to the virtual advisory board, wherein the data includes at least one of an agenda, an objective, and medical literature;
inviting, by the processor, a plurality of potential participants to join the virtual advisory board;
confirming, by the processor, attendance of actual participants who have joined the virtual advisory board;
presenting, by the processor, at least one vote or survey to the actual participants, and recording, by the processor, responses from the actual participants;
tracking, by the processor, usage data for the actual participants, wherein the usage data includes at least one of login times, login durations, cookies, and interactions with the data;
setting, by the processor, a compensation amount for each actual participant of the actual participants, wherein the compensation amount is based at least in part on the tracked usage data for the respective actual participant.

2. The method of claim 1, wherein the data further includes information regarding at least one of a pharmaceutical product, a medical device, a medical condition, a medical procedure, a treatment method, and a diagnostic method.

3. The method of claim 1, further comprising, determining, by the processor, participant information for each actual participant, wherein the participant information includes at least one of a name, position, title, location, educational credential, work experience, type of practice, prior medical advisory board participation, and salary.

4. The method of claim 3, wherein the compensation amount is also based at least in part on the participant information.

5. The method of claim 1, further comprising: preparing, by the processor, an advisory board resolution, where in the advisory board resolution includes at least the recorded responses to the at least one vote or survey.

6. The method of claim 1, wherein the actual participants are able to comment on the data.

7. The method of claim 6, wherein the comments are included in the usage data.

8. The method of claim 1, further comprising: providing, by the processor, an editable document, configured to track changes by the actual participants.

9. The method of claim 8, wherein changes are included in the usage data.

10. The method of claim 1, further comprising: preparing, by the processor, a final report including at least the data, the responses, and the usage data.

11. The method of claim 1, further comprising: receiving, by the processor, additional data from the actual participants, wherein the further data includes at least one of further medical literature, a document, and research data.

12. A computing system, comprising:
a processor;
a memory device coupled to the processor; and
a computer readable storage device coupled to the processor, wherein the storage device contains program code executable by the processor via the memory device to implement a method for providing a virtual advisory board, the method comprising:
storing, by a processor of a computing system, data related to the virtual advisory board, wherein the data includes at least one of an agenda, an objective, and medical literature;
inviting, by the processor, a plurality of potential participants to join the virtual advisory board;
confirming, by the processor, attendance of actual participants who have joined the virtual advisory board;
presenting, by the processor, at least one vote or survey to the actual participants, and recording, by the processor, responses from the actual participants;
tracking, by the processor, usage data for the actual participants, wherein the usage data includes at least one of login times, login durations, cookies, and interactions with the data;
setting, by the processor, a compensation amount for each actual participant of the actual participants, wherein the compensation amount is based at least in part on the tracked usage data for the respective actual participant.

13. The computing system of claim 12, wherein the method further comprises: determining, by the processor, participant information for each actual participant, wherein the participant information includes at least one of a name, position, title, location, educational credential, work experience, type of practice, prior medical advisory board participation, and salary.

14. The computing system of claim 12, wherein the method further comprises: preparing, by the processor, an advisory board resolution, where in the advisory board resolution includes at least the recorded responses to the at least one vote or survey.

15. The method of claim 1, further comprising, providing, by the processor, an editable document, configured to track changes by the actual participants.

16. The computing system of claim 12, wherein the method further comprises: preparing a final report including at least the data, the responses, and the usage data.

17. The computing system of claim 12, wherein the method further comprises: receiving, by the processor, additional data from the actual participants, wherein the further data includes at least one of further medical literature, a document, and research data.

18. A method of generating a report for a virtual advisory board, comprising:
receiving, by a processor of a computing system, data, wherein the data includes at least one of an agenda, an objective, and medical literature;
inviting, by the processor, a plurality of potential participants to join the virtual advisory board;
confirming, by the processor, attendance of actual participants who have joined the virtual advisory board;
providing, by the processor, actual participants with access to the data such that the actual participants are permitted to interact with the data, wherein interaction with the data includes at least one of commenting on, adding to, or editing the data;
tracking, by the processor, usage data for the actual participants, wherein the usage data includes at least one of login times, login durations, cookies, and interaction with the data;
presenting, by the processor, at least one vote or survey related to the data to the actual participants, and recording, by the processor, responses from the actual participants; and
generating, by the processor, the final report for the virtual advisory board, wherein the final report includes at least the data, a list of the actual participants, the usage data, and the recorded responses.

19. The method of claim 18, further comprising: setting, by the processor, a compensation amount for each actual participant of the actual participants, wherein the compensation amount is based at least in part on the tracked usage data for the respective actual participant.
